(19) Europäisches Patentamt
European Patent Office
Office européen des brevets

(11) EP 3 750 871 A1

(12) EUROPEAN PATENT APPLICATION
published in accordance with Art. 153(4) EPC

(43) Date of publication:
**16.12.2020 Bulletin 2020/51**

(21) Application number: **19750705.6**

(22) Date of filing: **05.02.2019**

(51) Int Cl.:
*C07C 253/20* (2006.01) *B01J 21/12* (2006.01)
*B01J 21/16* (2006.01) *B01J 23/08* (2006.01)
*B01J 23/20* (2006.01) *B01J 23/745* (2006.01)
*B01J 27/18* (2006.01) *B01J 29/08* (2006.01)
*B01J 29/40* (2006.01) *C07C 255/03* (2006.01)
*C07C 255/04* (2006.01) *C07C 255/08* (2006.01)
*C07C 255/50* (2006.01) *C07C 255/51* (2006.01)
*C07D 213/84* (2006.01) *C07B 61/00* (2006.01)

(86) International application number:
**PCT/JP2019/003933**

(87) International publication number:
**WO 2019/156036 (15.08.2019 Gazette 2019/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.02.2018 JP 2018019482**

(71) Applicant: **Toray Industries, Inc.**
**Tokyo, 103-8666 (JP)**

(72) Inventors:
- **TSUKAMOTO, Daijiro**
  **Kamakura-shi, Kanagawa 248-8555 (JP)**
- **KAWAMURA, Kenji**
  **Kamakura-shi, Kanagawa 248-8555 (JP)**
- **YAMADA, Katsushige**
  **Kamakura-shi, Kanagawa 248-8555 (JP)**
- **AKAHIRA, Masato**
  **Nagoya-shi, Aichi 455-8502 (JP)**
- **YAMAMOTO, Daisuke**
  **Nagoya-shi, Aichi 455-8502 (JP)**

(74) Representative: **Kador & Partner PartG mbB**
**Corneliusstraße 15**
**80469 München (DE)**

# (54) METHOD FOR PRODUCING NITRILE

(57) The present invention provides a method of producing a nitrile from a primary amide, characterized in that the primary amide is subjected to a dehydration reaction in a supercritical fluid in the presence of an acid catalyst. The present invention achieves the object of reducing the corrosion of a reactor and the thermal decomposition of raw materials, as well as provides the effect of improving the reaction rate and nitrile selectivity.

EP 3 750 871 A1

**Description**

Technical Field

The present invention relates to a method of producing a nitrile.

Background Art

Nitriles are chemical substances which are used in a number of applications, such as solvents, raw materials for pharmaceuticals, raw materials for agricultural chemicals, metal surface treatment agents, antioxidants and polymer raw materials.

Methods of dehydrating primary amides are widely used as methods of producing nitriles. Known specific examples of such methods include: a method of producing a nitrile from a primary amide, by carrying out the dehydration reaction in a pyridine-based solvent at a temperature of from room temperature to 50°C, using a dehydrating reagent such as diphosphorus pentaoxide, phosphoryl chloride, phosgene or a chloroformic acid ester (Patent Literature 1); a method of producing a nitrile from a primary amide, by carrying out the dehydration reaction in a mesitylene solvent at 165°C, using a rhenium compound as a catalyst (Patent Literature 2); a method of producing a nitrile from a primary amide, by carrying out the dehydration reaction in a phosphoric acid at 300°C, using phosphoric acid as a catalyst (Patent Literature 3); a method of producing a nitrile from a primary amide, by carrying out the dehydration reaction in a gas phase at a temperature of from 325°C to 345°C, using silicon dioxide as a catalyst (Patent Literature 4), and a method of producing a nitrile from a primary amide, by carrying out the dehydration reaction in the absence of a solvent at 250°C, using iron oxide, niobium oxide or the like as a catalyst (Patent Literature 5).

However, these conventional methods have been known to have problems such as, for example: one equivalent or more of a dehydrating reagent is required, and a harmful waste is produced (Patent Literature 1); it is necessary to use rhenium, which is extremely rare, and thus is not industrially suitable (Patent Literature 2); polyphosphoric acid generated from phosphoric acid precipitates in a reaction vessel, which makes it difficult to separate the resulting product and causes the occurrence of corrosion in the reactor (Patent Literature 3); heating to a high temperature is required in order to vaporize the amide raw material, which causes the thermal decomposition of the raw material (Patent Literature 4); and the method cannot be used for an amide having a high melting point, since the reaction is carried out in a molten amide (Patent Literature 5).

Examples of techniques which are expected to solve the above mentioned problems include performing an acid catalyst reaction in a supercritical fluid (Non-patent Documents 1 and 2). However, it is known that, when an acid catalyst reaction such as alkylation, amination, cracking, disproportionation, esterification, Fischer-Tropsch synthesis or isomerization is carried out in a supercritical fluid, there are cases where: only the reaction rate is improved; only the reaction selectivity is improved; both the reaction rate and the reaction selectivity are improved; and both the reaction rate and the reaction selectivity are not improved (Non-patent Document 1). Further, in a dehydration reaction of an alcohol in a supercritical fluid or a non-supercritical fluid, in the presence of an acid catalyst, both the reaction rate and the reaction selectivity are known to be higher in the non-supercritical fluid than in the supercritical fluid (Non-patent Document 2). Thus, technical effects of performing an acid catalyst reaction in a supercritical fluid are not uniform from the viewpoint of improving the reaction rate and the reaction selectivity.

Further, Non-patent Document 3 discloses a method of synthesizing a benzonitrile by dehydrating benzamide, which is a primary amide, in acetonitrile in the absence of an acid catalyst. Since the reaction temperature and the reaction pressure in this method are 350°C and 6.5 MPa, respectively, which are higher than the critical temperature and the critical pressure of acetonitrile (275°C and 4.8 MPa, respectively), it is thought that acetonitrile is in a supercritical state. However, it is described therein that the dehydration reaction of benzamide proceeds sufficiently even at 250°C, which is a temperature significantly lower than the above described reaction temperature (350°C) and which is equal to or lower than the critical temperature (275°C) of acetonitrile. Therefore, it is recognized that there is no technical effect in performing the dehydration reaction of a primary amide in a supercritical fluid, from the viewpoint of improving the reaction rate and the reaction selectivity.

Citation List

Patent Literatures

Patent Literature 1: JP 3185482 B
Patent Literature 2: JP 4190192 B

Patent Literature 3: US Patent No. 3297740
Patent Literature 4: US Patent No. 2144340
Patent Literature 5: JP 2016-513119 A

Non-patent Literatures

Non-patent Literature 1: Chemical Reviews, vol. 99, p. 453 to 473 (1999).
Non-patent Literature 2: Catalysis Today, vol. 121, p. 121 to 129 (2007).
Non-patent Literature 3: Journal of Organic Chemistry, vol. 78, p. 10567 to 10571 (2013).

Summary of Invention

Technical Problem

An object of the present invention is to solve the above mentioned problems associated with conventional methods of producing nitriles, and to provide a method of producing a nitrile which method has an excellent reaction rate and reaction selectivity.

Solution to Problem

As a result of intensive studies to solve the above mentioned problems, the present inventors have focused on the acid catalyst reaction in a supercritical fluid. Conventionally, it has been recognized by those skilled in the art, as technical common sense, that technical effects of performing an acid catalyst reaction in a supercritical fluid are not uniform from the viewpoint of improving the reaction rate and the reaction selectivity, and that the reaction rate and the reaction selectivity in the dehydration reaction of a primary amide in a supercritical fluid, in the absence of an acid catalyst, are not superior to those in a non-supercritical fluid. However, the present inventors have found out that, when the dehydration reaction of a primary amide in the presence of an acid catalyst is carried out in a supercritical fluid, the reaction rate and the nitrile selectivity can be markedly improved as compared to the corresponding reaction in a non-supercritical fluid, thereby completing the present invention.

That is, the present invention has the following constitutions (1) to (6).

(1) A method of producing a nitrile, the method including subjecting a primary amide to a dehydration reaction in a supercritical fluid in the presence of an acid catalyst.
(2) The method according to (1), wherein the primary amide is a primary amide containing one or two primary amide groups.
(3) The method according to (1) or (2), wherein the primary amide is a saturated alkylamide, an unsaturated alkylamide, or an amide containing an aromatic ring and/or a heterocyclic ring.
(4) The method according to any one of (1) to (3), wherein the acid catalyst is a solid acid catalyst.
(5) The method according to any one of (1) to (4), wherein the supercritical fluid is a supercritical fluid of one or more substances selected from the group consisting of carbon dioxide, an alcohol compound, an ether compound and a hydrocarbon compound.
(6) The method according to any one of (1) to (5), wherein the supercritical fluid is a supercritical fluid of one or more substances selected from the group consisting of carbon dioxide, methanol, ethanol, 1-propanol, 2-propanol, 1-butanol, 2-butanol, isobutanol, *tert*-butanol, *tert*-amyl alcohol, diethyl ether, methyl-*tert*-butyl ether, diisopropyl ether, 1,2-dimethoxyethane, tetrahydrofuran, pentane, hexane and benzene.

Advantageous Effects of Invention

According to the present invention, it is possible to enhance the reaction rate and the nitrile selectivity in a method of producing a nitrile by the dehydration of a primary amide.

Description of Embodiments

The present invention will now be described in further detail.

[Primary Amide]

In the present invention, the term "primary amide" refers to an organic compound containing one or more primary amide groups ($-CONH_2$). Any primary amide can be used as a raw material in the method of producing a nitrile according to the present invention, without particular limitations.

The number of primary amide groups contained in the primary amide is not particularly limited. However, a primary amide containing one or two primary amide groups can be preferably used.

The primary amide to be used in the present invention is not particularly limited. However, a saturated alkylamide, an unsaturated alkylamide, or an amide containing an aromatic ring and/or a heterocyclic ring can be preferably used.

The term "saturated alkylamide" refers to a primary amide which does not contain an unsaturated alkyl group, an aromatic ring and a heterocyclic ring, and which contains a saturated alkyl group having an arbitrary number of carbon atoms and having an arbitrary functional group(s). Examples of the saturated alkylamide include acetamide, propanamide, butylamide, valeramide, hexanamide, cyclohexanamide, malonamide, succinamide, glutaramide, adipamide, suberonamide, 1,3-cyclohexanediamide and 1,4-cyclohexanediamide.

The term "unsaturated alkylamide" refers to a primary amide which does not contain an aromatic ring and a heterocyclic ring, and which contains an unsaturated alkyl group having an arbitrary number of carbon atoms and having an arbitrary functional group(s). Examples of the unsaturated alkylamide include acrylamide, methacrylamide, butenamide, hexenamide, 2-hexene-1,6-diamide, 3-hexene-1,6-diamide and 2,4-hexadiene-1,6-diamide.

The term "amide containing an aromatic ring and/or a heterocyclic ring" refers to a primary amide containing an aromatic ring and/or a heterocyclic ring, and having an arbitrary number of carbon atoms and having an arbitrary functional group(s). Examples of the amide containing an aromatic ring and/or a heterocyclic ring include benzamide, 4-aminobenzamide, 2-hydroxybenzamide, isophthalamide, terephthalamide, cinnamyl amide, nicotinamide and isonicotinamide.

The term "arbitrary functional group" as used herein refers to an alkyl group, a carbonyl group (-CO-), a hydroxy group (-OH), an aldehyde group (-CHO), a carboxy group (-COOH), an ether bond (-O-), an ester bond (-COO-), an amino group ($-NH_2$), a nitro group ($-NO_2$), a cyano group (-C≡N), a secondary amide group (-CONHRi: wherein $R_1$ represents an arbitrary alkyl group), a tertiary amide group ($-CONR_1R_2$: wherein each of $R_1$ and $R_2$ represents an arbitrary alkyl group), a sulfo group ($-SO_3H$) or a halogen atom (-F, -Cl, -Br, -I).

Primary amides can be produced chemically and/or biologically, using various types of carbon sources, such as crude oil, charcoal, natural gases, biomass, exhaust gases and plastic products, as raw materials, and any of the thus produced primary amides can be used in the present invention.

In particular, as shown in the following scheme 1, a primary amide is industrially synthesized by heating a carboxylic acid (R-COOH) and ammonia to produce a carboxylic acid ammonium salt, followed by the dehydration of the carboxylic acid ammonium salt (disclosed, for example, in JP 2016-513119 A). Alternatively, as shown in scheme 2, a primary amide is industrially synthesized also by the ammonolysis of a carboxylic acid ester (R-COO-R'), (disclosed, for example, in JP 10-195035 A).

[Chem. 1]

Scheme 1

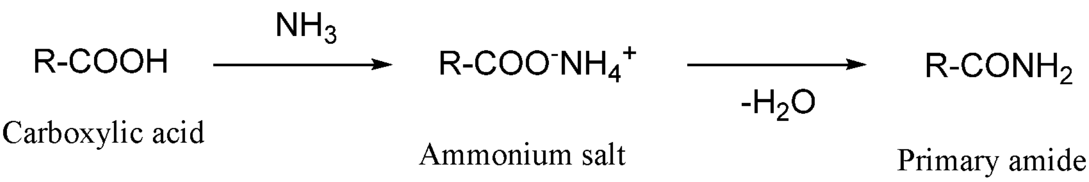


Carboxylic acid
Ammonium salt
Primary amide

Scheme 2

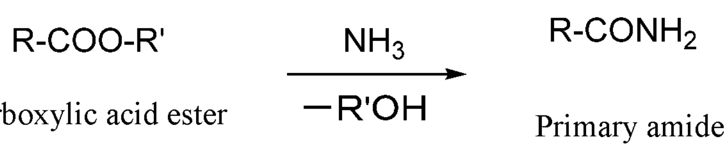


Carboxylic acid ester
Primary amide

In the present invention, a primary amide produced from a carboxylic acid or a carboxylic acid ester as described

above can also be used as a raw material, to produce a corresponding nitrile.

[Nitrile]

The "nitrile" to be produced by the present invention is an organic compound containing a cyano group (-C≡N), and produced by dehydrating some or all of the primary amide groups contained in the above described primary amide.

[Acid Catalyst]

The dehydration reaction of a primary amide is accelerated in the presence of an acid catalyst. In the present invention, the "acid catalyst" may be an acid catalyst which exhibits either a Bronsted acidity or a Lewis acidity. The acid catalyst is, for example, an inorganic acid, an organic acid or a solid acid catalyst, and a solid acid catalyst is preferably used in the present invention.

Examples of the inorganic acid which can be used in the present invention include: mineral acids such as sulfuric acid, hydrochloric acid, nitric acid, phosphoric acid and boric acid; halides containing one or more metal elements selected from the group consisting of Sn, Ga, In, Sc, Fe, Al, B, Zn, Ti, Zr, Sb and Cd; sulfate salts; hydrochloride salts; and nitrate salts.

Examples of the organic acid which can be used in the present invention include: organic sulfonic acids such as methanesulfonic acid, paratoluenesulfonic acid and paraaminobenzenesulfonic acid; and carboxylic acids such as formic acid, acetic acid and fluoroacetic acid.

The term "solid acid catalyst" refers to an acid catalyst which is a solid having an acidic active site on the surface thereof. The solid acid catalyst which can be used in the present invention may be, for example, a metal oxide, a metal sulfide, a zeolite, a clay, an acidic ion exchange resin, a heteropoly acid, a solid phosphoric acid or hydroxyapatite.

Examples of the metal oxide which serves as the solid acid catalyst include oxides containing one or more metal elements selected from the group consisting of Sc, Y, Ce, Ti, Zr, V, Nb, Ta, Cr, Mo, W, Mn, Fe, Zn, Cd, Al, Ga, In, Si, Ge, Sn and Pb. More specific examples thereof include scandium oxide ($SC_2O_3$), cerium oxide ($CeO_2$), anatase-type titanium oxide (A-$TiO_2$), rutile-type titanium oxide (R-$TiO_2$), zirconium oxide ($ZrO_2$), vanadium oxide ($V_2O_5$), niobium oxide ($NbiO_5$), tantalum oxide ($Ta_2O_5$), chromium oxide ($Cr_2O_3$), molybdenum oxide ($MoO_3$), tungsten oxide ($WO_3$), manganese oxide ($MnO_2$), iron oxides ($Fe_2O_3$, $Fe_3O_4$), zinc oxide (ZnO), aluminum oxide ($Al_2O_3$), gallium oxide ($Ga_2O_3$), indium oxide ($In_2O_3$), silicon dioxide ($SiO_2$), germanium oxide ($GeO_2$), tin oxide ($SnO_2$) and lead oxide (PbO).

Examples of the zeolite which serves as the solid acid catalyst include zeolites to which structural codes composed of three alphabet letters are assigned in the database of International Zeolite Association. Specific examples thereof include zeolites to which structural codes such as LTA, FER, MWW, MFI, MOR, LTL, FAU, BEA, CHA and CON are assigned.

Examples of the clay which serves as the solid acid catalyst include kaolin, montmorillonite, bentonite, saponite and acid earth.

Examples of the acidic ion exchange resin which serves as the solid acid catalyst include styrene-based sulfonic acid-type ion exchange resins and phenol-based sulfonic acid-type ion exchange resins. Specific examples thereof include: DIAION manufactured by Mitsubishi Chemical Corporation; Lewatit manufactured by Lanxess Inc.; Amberlite and Amberlyst manufactured by Rohm and Haas Company; and DOWEX manufactured by Dow Inc.

The heteropoly acid is an acid catalyst composed of a hetero element such as Si, P or As, a poly element such as Mo, W or V, and oxygen. Examples of the heteropoly acid which serves as the solid acid catalyst include phosphotungstic acid, silicotungstic acid, phosphomolybdic acid and silicomolybdic acid.

Examples of the solid phosphoric acid which serves as the solid acid catalyst include phosphate salts containing one or more metal elements selected from the group consisting of Si, B, Al, Zr, Zn, Ti and Fe. More specific examples thereof include silicon phosphate, boron phosphate, aluminum phosphate, zirconium phosphate, zinc phosphate, titanium phosphate and iron phosphate.

As the acid catalyst, two or more kinds of acid catalysts arbitrarily selected from the above described acid catalysts can be mixed and used as a mixture, or alternatively, two or more kinds of acid catalysts can be chemically compounded and used as a composite. For example, silicon dioxide supporting phosphoric acid, silicon dioxide supporting a metal halide, silica-alumina ($SiO_2$-$Al_2O_3$) which is a composite of silicon dioxide and aluminum oxide, and the like, are also included in the solid acid catalysts which can be used in the present invention.

[Supercritical Fluid]

The term "supercritical fluid" refers to a substance at a temperature and a pressure equal to or higher than the critical point (supercritical state). In the present invention, the substance as the original material of the supercritical fluid is not particularly limited, and a supercritical fluid of any substance can be used as a reaction field for the dehydration

reaction of the primary amide to be used in the present invention, as long as the effects of the present invention are achieved. Further, it is possible to use a supercritical fluid of one kind of substance, or a supercritical fluid of a mixture of two or more kinds of different substances, as the supercritical fluid to be used in the present invention.

The substance which can be used as the supercritical fluid may be, for example, ammonia, carbon monoxide, carbon dioxide, water, an alcohol compound, an ether compound, an ester compound, a hydrocarbon compound or an organochlorine compound. The substance may also be a mixture of two or more kinds of these substances.

Examples of the alcohol compound include methanol, ethanol, 1-propanol, 2-propanol, 1-butanol, 2-butanol, isobutanol, *tert*-butanol, *tert*-amyl alcohol and cyclohexanol.

Examples of the ether compound include dimethyl ether, diethyl ether, methyl-*tert*-butyl ether, diisopropyl ether, dibutyl ether, 1,2-dimethoxyethane, tetrahydrofuran, 1,4-dioxane and cyclopentyl methyl ether.

Examples of the ester compound include methyl formate, ethyl formate, methyl acetate, ethyl acetate, n-propyl acetate and n-butyl acetate.

Examples of the hydrocarbon compound include ethane, ethylene, propane, propylene, butane, isobutane, pentane, hexane, heptane, octane, decane, cycloheptane, cyclohexane, benzene, toluene and xylene.

Examples of the organochlorine compound include carbon tetrachloride, dichloromethane and chloroform.

Among these, preferably used are: alcohol compounds such as carbon dioxide, methanol, ethanol, 1-propanol, 2-propanol, 1-butanol, 2-butanol, isobutanol, *tert*-butanol, *tert*-amyl alcohol, and cyclohexanol; ether compounds such as dimethyl ether, diethyl ether, methyl-*tert*-butyl ether, diisopropyl ether, dibutyl ether, 1,2-dimethoxyethane, diglyme, tetrahydrofuran, 1,4-dioxane and cyclopentyl methyl ether; and hydrocarbon compounds such as ethane, ethylene, propane, propylene, butane, isobutane, pentane, hexane, heptane, octane, decane, cycloheptane, cyclohexane, benzene, toluene and xylene. More preferably used are methanol, ethanol, 1-propanol, 2-propanol, 1-butanol, 2-butanol, isobutanol, *tert*-butanol, *tert*-amyl alcohol, diethyl ether, methyl-*tert*-butyl ether, diisopropyl ether, 1,2-dimethoxyethane, tetrahydrofuran, pentane, hexane and benzene.

The critical temperature and the critical pressure of substances are described in CRC Handbook of Chemistry and Physics, The 84th edition, CRC Press, 2003-2004, page 1023 to 1038. The critical temperature and the critical pressure of typical substances are shown in Table 1.

[Table 1]

Table 1 Critical temperature and critical pressure of substances

| Substance | Critical temperature $T_c$ (°C) | Critical pressure $P_c$ (MPa) |
|---|---|---|
| Ammonia | 132 | 11.4 |
| Carbon monoxide | -140 | 3.5 |
| Carbon dioxide | 31 | 7.4 |
| Ethane | 32 | 4.9 |
| Propane | 97 | 4.2 |
| Isobutane | 135 | 3.6 |
| Water | 374 | 22.1 |
| Methanol | 240 | 8.1 |
| Ethanol | 241 | 6.1 |
| 1-Propanol | 264 | 5.2 |
| 2-Propanol | 235 | 4.8 |
| 1-Butanol | 290 | 4.4 |
| 2-Butanol | 263 | 4.2 |
| Isobutanol | 275 | 4.3 |
| *Tert*-butanol | 233 | 4.0 |
| *Tert*-amyl alcohol | 271 | 3.7 |
| Diethyl ether | 194 | 3.6 |

(continued)

| Substance | Critical temperature $T_c$ (°C) | Critical pressure $P_c$ (MPa) |
|---|---|---|
| Methyl-*tert*-butyl ether | 224 | 3.4 |
| Diisopropyl ether | 227 | 2.8 |
| 1,2-Dimethoxyethane | 267 | 3.9 |
| Tetrahydrofuran | 267 | 5.2 |
| Pentane | 197 | 3.4 |
| Hexane | 234 | 3.0 |
| Benzene | 289 | 4.9 |

[Reaction Method]

The production method according to the present invention can be carried out in a reaction method using any of a batch tank reactor, a semi-batch tank reactor, a continuous tank reactor and a continuous tubular reactor.

In the case of carrying out the reaction using a solid acid catalyst, the reaction can be carried out by any of a suspended bed system, a fixed bed system, a moving bed system and a fluidized bed system.

[Reaction Temperature]

The reaction temperature in the dehydration reaction of a primary amide is not particularly limited, as long as it is equal to or higher than the critical temperature of the substance to be used as the supercritical fluid. The reaction temperature is preferably 300°C or lower, because it allows for reducing the thermal decomposition of the primary amide as the raw material.

[Reaction Pressure]

The reaction pressure in the dehydration reaction is not particularly limited, as long as it is equal to or higher than the critical pressure of the substance to be used as the supercritical fluid.

In any of the cases of the batch tank reactor, the semi-batch tank reactor, the continuous tank reactor and the continuous tubular reactor, the pressure inside the reactor can be increased by using an arbitrary pump. For example, a pump for feeding the raw material to the reactor can be used to apply a pressure from the raw material feed port side of the reactor. The pressure inside the reactor can be controlled by providing a back pressure valve to the discharge side of the reactor.

In the case of using a tank reactor, when a sufficient amount of a substance that is a solid or a liquid in a standard state (25°C, atmospheric pressure) is introduced into the reactor, and the reactor is heated in a non-open state, the vapor pressure of the substance corresponding to the temperature is applied to the reactor. Accordingly, in cases where the temperature of the reactor is adjusted to equal to or higher than the critical temperature of the substance, the pressure inside the reactor is naturally increased to equal to or higher than the critical pressure. In this case, there is no need to apply a pressure from the outside in order to achieve the critical pressure. It is noted that the "sufficient amount" as used herein refers to the amount of a substance which is sufficient enough so that all of the substance does not vaporize when the critical temperature is reached.

The substance to be used as the supercritical fluid in the reactor can be controlled to a supercritical state, for example, by a method in which a sufficient amount of a substance that is a solid or a liquid in a standard state (25°C, atmospheric pressure) is introduced into the reactor, and the reactor is heated in a non-open state to a temperature equal to or higher than the critical temperature of the substance, as described above, thereby controlling the interior of the reactor to a temperature equal to or higher than the critical temperature and to a pressure equal to or higher than the critical pressure. A method can also be used in which the pressure inside the reactor is increased to a pressure equal to or higher than the critical pressure of the substance, using a pump or the like, and then the temperature inside the reactor is increased to a temperature equal to or higher than the critical temperature of the substance. Further, a method can also be used in which the temperature inside the reactor is increased to a temperature equal to or higher than the critical temperature, and then the internal pressure of the reactor is increased to a pressure equal to or higher than the critical pressure, using a pump or the like.

[Atmosphere]

In the reactor for carrying out the dehydration reaction of a primary amide, a substance that is a gas in a standard state (25°C, atmospheric pressure), such as nitrogen, helium, argon, oxygen, hydrogen, ammonia or the like, may coexist with the supercritical fluid. These gases are capable of forming a good mixed state with the supercritical fluid in the reactor. Above all, coexistence of ammonia is preferred, since ammonia is expected to be capable of reducing the generation of a carboxylic acid due to the deamidation of the primary amide. Further, in order to reduce the reverse reaction to the primary amide due to the hydration of the produced nitrile, it is preferred that water vapor is absent or present in a minimum amount.

[Recovery of Nitrile]

The nitrile produced by the method of producing a nitrile according to the present invention can be recovered by carrying out general separation and purification operations, such as filtration, extraction, distillation, crystallization and recrystallization, after the completion of the reaction.

[Production of Hexamethylenediamine]

The method of producing a nitrile according to the present invention is capable of producing adiponitrile, when adipamide is used as the primary amide. The resulting adiponitrile can be hydrogenated by a known method (such as one disclosed in JP 2000-508305 A) to produce hexamethylenediamine, which is the raw material of polyamide 6,6.

Examples

The present invention will now be described in further detail with reference to Examples. It is noted, however, that the present invention is in no way limited to the following Examples. The reaction results in Examples and Comparative Examples are defined by the following equations.

$$\text{Proportion of dehydrated primary amide groups (\%)} = (\text{amount of dehydrated primary amide groups} / \text{total amount of primary amide groups in raw material}) \times 100.$$

$$\text{Nitrile selectivity (\%)} = \text{amount of nitrile produced (mol)} / (\text{amount of raw material amide (mol)} - \text{amount of unreacted amide (mol)}) \times 100.$$

The resulting reaction solution and an aqueous solution of a concentrate of the reaction solution were analyzed by gas chromatography (GC) and high-speed liquid chromatography (HPLC), respectively. The quantification of the resulting product was carried out based on an absolute calibration curve prepared using a reference standard. The quantitative analysis of each nitrile was mainly carried out by GC, and the quantitative analysis of each amide was mainly carried out by HPLC. The analysis conditions for GC and HPLC are shown below.

[GC Analysis Conditions]

GC apparatus: GC2010 plus (manufactured by Shimadzu Corporation)
Column: InertCap for amines; length: 30 m; inner diameter: 0.32 mm (manufactured by GL Sciences Inc.)
Carrier gas: helium; linear velocity: constant (40.0 cm/sec)
Vaporization chamber temperature: 250°C
Detector temperature: 250°C
Column oven temperature: 100°C → (10°C/min) → 230°C, 10 min (total: 23 min)
Detector: FID

[HPLC Analysis Conditions]

HPLC apparatus: Prominence (manufactured by Shimadzu Corporation)
Column: Synergi hydro-RP (manufactured by Phenomenex Inc.); length: 250 mm;
inner diameter: 4.60 mm; particle size: 4 μm
Mobile phase: 0.1% by weight aqueous phosphoric acid solution/ acetonitrile = 95/5 (volume ratio)
Flow velocity: 1.0 mL/min
Detector: UV (210 nm)
Column temperature: 40°C.

(Example 1) Production of Nitrile from Adipamide

Into a stainless steel autoclave (manufactured by Taiatsu Techno Corporation) having a capacity of 0.1 L, 0.144 g of adipamide (manufactured by Tokyo Chemical Industry Co., Ltd.), 50 mL of *tert*-butanol (manufactured by Wako Pure Chemical Industries, Ltd.), and 0.05 g of an α-iron oxide (α-$Fe_2O_3$; manufactured by Wako Pure Chemical Industries, Ltd.) as a catalyst were introduced. The interior of the autoclave was adjusted to 30°C, and the interior of the autoclave was purged with nitrogen while stirring at a stirring rate of 500 rpm. Subsequently, ammonia gas was added such that the partial pressure of ammonia gas inside the autoclave was adjusted to 0.18 MPa, and the autoclave was maintained for 30 minutes thereafter. Nitrogen was then added to the autoclave, while continuing the stirring, such that the partial pressure of nitrogen inside the autoclave was adjusted to 0.32 MPa (total pressure (gauge pressure): 0.50 MPa). Thereafter, the temperature inside the autoclave was raised to 235°C. The gauge pressure at 235°C was 4.8 MPa. The autoclave was maintained for one hour at 235°C, and then allowed to cool to room temperature. After releasing the gas in the autoclave so as to reduce the pressure inside the autoclave to normal pressure, the reaction solution was recovered. The catalyst was removed by filtration, and the resulting supernatant was analyzed by GC. Further, the supernatant was concentrated by a rotatory evaporator (manufactured by Tokyo Rikakikai Co., Ltd.) to obtain a concentrate, an aqueous solution of the concentrate was prepared, and then analyzed by HPLC. The results are shown in Table 2.

(Comparative Example 1)

The reaction was carried out in the same manner as in Example 1, except that no catalyst was used. The results are shown in Table 2.

(Comparative Example 2)

The reaction was carried out in the same manner as in Example 1, except that the temperature inside the autoclave was adjusted to 225°C. The results are shown in Table 2.

(Comparative Example 3)

The reaction was carried out in the same manner as in Example 1, except that the temperature inside the autoclave was adjusted to 230°C. The results are shown in Table 2.

(Example 2)

The reaction was carried out in the same manner as in Example 1, except that 2-propanol (manufactured by Nacalai Tesque Inc.) was used instead of *tert*-butanol, and that the temperature inside the autoclave was adjusted to 237°C. The results are shown in Table 2.

(Example 3)

The reaction was carried out in the same manner as in Example 2, except that the temperature inside the autoclave was adjusted to 240°C. The results are shown in Table 2.

(Comparative Example 4)

The reaction was carried out in the same manner as in Example 2, except that the temperature inside the

autoclave was adjusted to 230°C. The results are shown in Table 2.

(Comparative Example 5)

The reaction was carried out in the same manner as in Example 2, except that the temperature inside the autoclave was adjusted to 232°C. The results are shown in Table 2.

(Example 4)

The reaction was carried out in the same manner as in Example 1, except that diisopropyl ether was used instead of *tert*-butanol, and that the temperature inside the autoclave was adjusted to 230°C. The results are shown in Table 2.

(Comparative Example 6)

The reaction was carried out in the same manner as in Example 4, except that the temperature inside the autoclave was adjusted to 225°C. The results are shown in Table 2.

[Table 2]

Table 2: Production of nitrile from adipamide

| | Raw material | Catalyst | Substance used as reaction field | Reaction temperature (°C) | Reactor internal pressure (MPa) | State | Proportion of dehydrated primary amide groups (%) | Nitrile selectivity (%) |
|---|---|---|---|---|---|---|---|---|
| Example 1 | adipamide | $Fe_2O_3$ | *tert*-butanol | 235 | 4.8 | Supercritical | 13.8 | 80.7 |
| Comparative Example 1 | adipamide | None | *tert*-butanol | 235 | 4.8 | Supercritical | 0.5 | 9.8 |
| Comparative Example 2 | adipamide | $Fe_2O_3$ | *tert*-butanol | 225 | 3.9 | Non-supercritical | 0.6 | 20.1 |
| Comparative Example 3 | adipamide | $Fe_2O_3$ | *tert*-butanol | 230 | 4.3 | Non-supercritical | 0.9 | 24.2 |
| Example 2 | adipamide | $Fe_2O_3$ | 2-propanol | 237 | 5.6 | Supercritical | 52.2 | 94.4 |
| Example 3 | adipamide | $Fe_2O_3$ | 2-propanol | 240 | 6.0 | Supercritical | 56.8 | 86.7 |
| Comparative Example 4 | adipamide | $Fe_2O_3$ | 2-propanol | 230 | 4.4 | Non-supercritical | 2.5 | 32.0 |
| Comparative Example 5 | adipamide | $Fe_2O_3$ | 2-propanol | 232 | 5.0 | Non-supercritical | 4.0 | 55.0 |
| Example 4 | adipamide | $Fe_2O_3$ | diisopropyl ether | 230 | 3.0 | Supercritical | 19.8 | 81.2 |
| Comparative Example 6 | adipamide | $Fe_2O_3$ | diisopropyl ether | 225 | 2.6 | Non-supercritical | 13.0 | 60.2 |

(Example 5) Production of Benzonitrile from Benzamide

The reaction was carried out in the same manner as in Example 1, except that 0.121 g of benzamide (manufactured by Wako Pure Chemical Industries, Ltd.) was used instead of adipamide. After adding 2-propanol to the recovered reaction solution and stirring the mixture at room temperature, the catalyst was removed by centrifugation, and the supernatant was analyzed by GC. The results are shown in Table 3.

(Example 6) Production of Nicotinonitrile from Nicotinamide

The reaction was carried out in the same manner as in Example 1, except that 0.122 g of nicotinamide (manufactured by Wako Pure Chemical Industries, Ltd.) was used instead of adipamide. The results are shown in Table 3.

(Example 7) Production of Nitrile from Terephthalamide

The reaction was carried out in the same manner as in Example 1, except that 0.164 g of terephthalamide (manufactured by Tokyo Chemical Industry Co., Ltd.) was used instead of adipamide. The recovered reaction solution was concentrated by a rotatory evaporator to obtain a concentrate, and a solution obtained by dissolving the concentrate in dimethyl sulfoxide (DMSO: manufactured by Wako Pure Chemical Industries, Ltd.) was analyzed by HPLC. The results are shown in Table 3.

(Example 8) Production of Benzonitrile from Benzamide

The reaction was carried out in the same manner as in Example 5, except that hexane (manufactured by Wako Pure Chemical Industries, Ltd.) was used instead of *tert*-butanol, and that the reaction temperature was adjusted to 237°C. The results are shown in Table 3.

(Example 9) Production of Acrylonitrile from Acrylamide

The reaction was carried out in the same manner as in Example 5, except that acrylamide (manufactured by Wako Pure Chemical Industries, Ltd.) was used instead of benzamide. The results are shown in Table 3.

(Example 10) Production of Hexanenitrile from Hexanamide

The reaction was carried out in the same manner as in Example 1, except that 0.115 g of hexaneamide (manufactured by Sigma-Aldrich Co. LLC.) was used instead of adipamide. The catalyst was removed by centrifugation, and the supernatant was analyzed by GC. The results are shown in Table 3.

(Examples 11 to 17) Production of Hexanenitrile from Hexanamide

In each of the Examples, the reaction was carried out in the same manner as in Example 10, except that, instead of using the α-iron oxide ($Fe_2O_3$), niobium oxide ($Nb_2O_5$; manufactured by Wako Pure Chemical Industries, Ltd.), indium oxide ($In_2O_3$; manufactured by Wako Pure Chemical Industries, Ltd.), an MFI zeolite (ZSM-5; manufactured by JGC C&C), a FAU zeolite (JRC-Z-HY; manufactured by JGC C&C, provided by Reference Catalyst Committee, Catalysis Society of Japan), montmorillonite (manufactured by Sigma-Aldrich Co. LLC.), aluminum phosphate (manufactured by Sigma-Aldrich Co. LLC.) or silica-alumina (manufactured by JGC C&C) was used. The results are shown in Table 3.

[Table 3]

Table 3: Production of nitriles from various kinds of primary amides

| | Raw material | Catalyst | Substance used as reaction field | Reaction temperature (°C) | Reactor internal pressure (MPa) | State | Proportion of dehydrated primary amide groups (%) | Nitrile selectivity (%) |
|---|---|---|---|---|---|---|---|---|
| Example 5 | benzamide | $Fe_2O_3$ | *tert*-butanol | 235 | 5.4 | Supercritical | 16.5 | 78.0 |
| Example 6 | nicotinamide | $Fe_2O_3$ | *tert*-butanol | 235 | 5.1 | Supercritical | 15.7 | 81.6 |
| Example 7 | terephthalamide | $Fe_2O_3$ | *tert*-butanol | 235 | 5.6 | Supercritical | 8.9 | > 99.9 |
| Example 8 | benzamide | $Fe_2O_3$ | hexane | 237 | 3.2 | Supercritical | 41.3 | 90.6 |
| Example 9 | acrylamide | $Fe_2O_3$ | hexane | 237 | 3.2 | Supercritical | 13.1 | 90.1 |
| Example 10 | hexanamide | $Fe_2O_3$ | *tert*-butanol | 235 | 6.1 | Supercritical | 20.4 | 79.7 |
| Example 11 | hexanamide | $Nb_2O_5$ | *tert*-butanol | 235 | 5.9 | Supercritical | 19.5 | 82.3 |
| Example 12 | hexanamide | $In_2O_3$ | *tert*-butanol | 235 | 5.9 | Supercritical | 22.2 | 83.4 |
| Example 13 | hexanamide | MFI zeolite | *tert*-butanol | 235 | 5.9 | Supercritical | 13.2 | 75.4 |
| Example 14 | hexanamide | FAU zeolite | *tert*-butanol | 235 | 5.8 | Supercritical | 11.5 | 72.9 |
| Example 15 | hexanamide | Montmorillonite | *tert*-butanol | 235 | 5.7 | Supercritical | 26.0 | 80.2 |
| Example 16 | hexanamide | Aluminum phosphate | *tert*-butanol | 235 | 5.8 | Supercritical | 17.4 | 70.5 |
| Example 17 | hexanamide | Silica-alumina | *tert*-butanol | 235 | 5.9 | Supercritical | 12.5 | 74.8 |

In a supercritical fluid, the proportion of dehydrated primary amide groups is markedly high, and thus it can be seen that the reaction rate of the dehydration reaction has been improved. Further, it can be seen that the nitrile selectivity is markedly high in a supercritical fluid. Therefore, the results of Examples 1 to 4 and Comparative Examples 1 to 6 have shown that, by carrying the dehydration reaction of a primary amide out in a supercritical fluid in the presence of an acid catalyst, it is possible to improve the reaction rate and to produce a nitrile at a high nitrile selectivity.

Further, the results of Examples 5 to 17 have shown that the present invention can be used for the production of nitriles from various kinds of primary amides, in various kinds of supercritical fluids, and in the presence of various kinds of acid catalysts.

Industrial Applicability

The method of producing a nitrile according to the present invention provides an excellent reaction rate and nitrile selectivity, and the nitriles obtained by the present method can be suitably used in a number of applications, such as solvents, raw materials for pharmaceuticals, raw materials for agricultural chemicals, metal surface treatment agents, antioxidants and polymer raw materials.

## Claims

1. A method of producing a nitrile, the method comprising subjecting a primary amide to a dehydration reaction in a supercritical fluid in the presence of an acid catalyst.

2. The method according to claim 1, wherein said primary amide is a primary amide comprising one or two primary amide groups.

3. The method according to claim 1 or 2, wherein said primary amide is a saturated alkylamide, an unsaturated alkylamide, or an amide comprising an aromatic ring and/or a heterocyclic ring.

4. The method according to any one of claims 1 to 3, wherein said acid catalyst is a solid acid catalyst.

5. The method according to any one of claims 1 to 4, wherein said supercritical fluid is a supercritical fluid of one or more substances selected from the group consisting of carbon dioxide, an alcohol compound, an ether compound and a hydrocarbon compound.

6. The method according to any one of claims 1 to 5, wherein said supercritical fluid is a supercritical fluid of one or more substances selected from the group consisting of carbon dioxide, methanol, ethanol, 1-propanol, 2-propanol, 1-butanol, 2-butanol, isobutanol, *tert*-butanol, *tert*-amyl alcohol, diethyl ether, methyl-*tert*-butyl ether, diisopropyl ether, 1,2-dimethoxyethane, tetrahydrofuran, pentane, hexane and benzene.

## INTERNATIONAL SEARCH REPORT

International application No. PCT/JP2019/003933

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl. C07C253/20, B01J21/12, B01J21/16, B01J23/08, B01J23/20, B01J23/745, B01J27/18, B01J29/08, B01J29/40, C07C255/03, C07C255/04, C07C255/08, C07C255/50, C07C255/51, C07D213/84, C07B61/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CASREACT (STN)

C. DOCUMENTS CONSIDERED TO BE RELEVANT

| Category* | Citation of document, with indication, where appropriate, of the relevant passages | Relevant to claim No. |
|---|---|---|
| X<br>A | NGUYEN, Thi Thu Hang et al., "Synthesis of Acetonitrile from Ammonium Acetate in Sub- and Supercritical Water", Chemie Ingenieur Technik, 2014, vol. 86, no. 1-2, pp. 161-168, ISSN 1522-2640, in particular, pp. 164-166, fig. 2, 7, 8 | 1-4<br>5-6 |
| A | JP 2016-216362 A (NIPPON STEEL & SUMITOMO METAL CORPORATION) 22 December 2016, claims, examples & WO 2015/099053 A1 | 1-6 |
| A | JP 2009-132663 A (NATIONAL INSTITUTE OF ADVANCED INDUSTRIAL SCIENCE AND TECHNOLOGY) 18 June 2009, claims, examples (Family: none) | 1-6 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 April 2019 (05.04.2019) | 23 April 2019 (23.04.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3185482 B
- JP 4190192 B
- US 3297740 A
- US 2144340 A
- JP 2016513119 A
- JP 10195035 A
- JP 2000508305 A


### Non-patent literature cited in the description


- *Chemical Reviews,* 1999, vol. 99, 453-473
- *Catalysis Today,* 2007, vol. 121, 121-129
- *Journal of Organic Chemistry,* 2013, vol. 78, 10567-10571
- CRC Handbook of Chemistry and Physics. CRC Press, 2003, vol. 2004, 1023-1038